(19) Europäisches Patentamt

European Patent Office

Office européen des brevets

(11) **EP 0 628 301 B1**

(12) **EUROPÄISCHE PATENTSCHRIFT**

(45) Veröffentlichungstag und Bekanntmachung des Hinweises auf die Patenterteilung:
**25.10.2000 Patentblatt 2000/43**

(51) Int. Cl.[7]: **A61K 7/09**

(21) Anmeldenummer: **94104920.7**

(22) Anmeldetag: **29.03.1994**

(54) **Mittel und Verfahren zur dauerhaften Haarverformung**

Composition and process for permanent waving of hair

Composition et procédé pour la déformation permanente de cheveux

(84) Benannte Vertragsstaaten:
**DE ES FR GB IT**

(30) Priorität: **27.05.1993 DE 4317663**

(43) Veröffentlichungstag der Anmeldung:
**14.12.1994 Patentblatt 1994/50**

(73) Patentinhaber:
**Wella Aktiengesellschaft**
**64274 Darmstadt (DE)**

(72) Erfinder:
- **Maresch, Gerhard**
  **D-64295 Darmstadt (DE)**
- **Burg, Wilfried**
  **D-64521 Gross-Gerau (DE)**

(56) Entgegenhaltungen:
**EP-A- 0 288 151**      **EP-A- 0 440 547**
**EP-A- 0 614 657**      **DE-A- 3 707 415**
**DE-A- 4 210 481**      **DE-A- 4 225 832**
**GB-A- 921 543**        **US-A- 3 847 165**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Xerox (UK) Business Services
2.16.7 (HRS)/3.6

**Beschreibung**

Die vorliegende Erfindung betrifft ein aus 2 Komponenten erhältliches Mittel zur dauerhaften Haarverformung sowie ein Verfahren zur dauerhaften Haarverformung unter Verwendung dieses Mittels.

Bekanntlich besteht die klassische Technik zur Durchführung der dauerhaften Haarverformung darin, daß in einer ersten Stufe die Disulfidbindungen des Haarkeratins mit Hilfe eines Mittels, welches einen reduzierenden Wirkstoff enthält, (Haarverformungsmittel) geöffnet werden, sodann das Haar in die gewünschte Form gebracht wird und anschließend die Disulfidbindungen unter Verwendung eines einen oxidierenden Wirkstoff enthaltenden Mittels (Fixiermittel) wiederverknüpft werden.

Als reduzierender Wirkstoff werden hierbei insbesondere anorganische Sulfite, Thioglykolsäure oder Thiomilchsäure, beziehungsweise deren Salze, Mercaptocarbonsäureester, Cystein und dessen Derivate oder Cysteamin und dessen Derivate verwendet.

Es ist bekannt, daß Mercaptocarbonsäureester, wie zum Beispiel der Thioglykolsäureglyzerinester, bezüglich ihrer Hautverträglichkeit sowie ihres Sensibilisierungsrisikos nicht zufriedenstellend sind, während die Thioglykolsäure enthaltenden Zubereitungen in der Regel einen wenig haut- und haarverträglichen pH-Wert von etwa 8 bis 9 aufweisen müssen, um eine ausreichende Verformungswirkung zu gewährleisten. Die Einstellung des für Thioglykolsäure enthaltende Haarverformungsmittel erforderlichen alkalischen pH-Wertes erfolgt hierbei durch Monoethanolamin oder eine Mischung aus Ammoniak und Ammoniumhydrogencarbonat.

Aufgrund der Instabilität der heute üblichen Puffersysteme im sauren bis schwach alkalischen pH-Bereich ist es auch bei Haarverformungsmitteln für geschädigtes, gefärbtes oder gebleichtes Haar nicht möglich, einen neutralen pH-Wert einzustellen, da der pH-Wert dieser Mittel aus Stabilitätsgründen mindestens bei 7,8 bis 8 liegen muß. Hierdurch wird das bereits vorgeschädigte Haar zusätzlich geschädigt.

In der nicht vorveröffentlichten älteren Patentanmeldung EP-A 0 614 657 wird ein Mittel zur dauerhaften Verformung von Haaren beschrieben, das Thioglykolsäure und/oder ein Salz derselben enthält und aus zwei bis zur Anwendung voneinander getrennt gehaltenen Zusammensetzungen besteht, wobei die Zusammensetzung (A) mindestens ein Aminosäurechlorid und mindestens ein Polyol bzw. dessen Methyl- oder Ethylether in wäßriger Lösung enthält und einen pH-Wert zwischen etwa 4,5 und 6,5 aufweist, und die zweite, davon getrennt gehaltene Zusammensetzung (B) Ammoniumhydrogencarbonat, Ammoniumcarbonat und/oder Ammoniumcarbamat enthält, wobei der pH-Wert zwischen etwa 8 und 9,5 liegt, und beide Zusammensetzungen unmittelbar vor der Anwendung auf menschliches Haar vermischt werden, und die dadurch hergestellte gebrauchsfertige Zusammensetzung(AB) einen pH-Wert zwischen 7 und 8 aufweist.

Es bestand daher die Aufgabe, ein Haarverformungsmittel zur Verfügung zu stellen, das keine sensibilisierenden Mercaptocarbonsäureester enthält und eine gleichmäßige und wirkungsvolle Umformung sowohl von geschädigtem als auch von normalem Haar im neutralen pH-Bereich (pH = 7 bis 7,5) ermöglicht.

Es wurde nunmehr gefunden, daß eine schonende und gleichzeitig wirkungsvolle Umformung der Haare möglich ist, wenn hierzu ein Mercaptocarbonsäureester-freies Mittel mit einem pH von 7 bis 7,5, welches unmittelbar vor der Anwendung aus 2 Komponenten hergestellt wird, verwendet wird.

Gegenstand der vorliegenden Erfindung ist daher ein Mittel zur dauerhaften Verformung von Haaren auf der Basis einer keratinreduzierenden Verbindung, welches frei ist von Mercaptocarbonsäureestern und einen pH-Wert von 7 bis 7,5 aufweist, dadurch gekennzeichnet, daß es unmittelbar vor der Anwendung durch Vermischen zweier Komponenten hergestellt wird, wobei die eine Komponente (Komponente A) die keratinreduzierende Verbindung enthält und einen pH-Wert von kleiner 7 aufweist, während die andere Komponente (Komponente B) ein Puffersystem enthält und einen pH-Wert von 8 bis 10 aufweist, ,wobei ein Mittel ausgenommen ist, bei dem die keratinreduzierende Verbindung Thioglykolsäure und/oder ein Salz derselben ist und gleichzeitig die Komponente A mindestens ein Aminosäurechlorid und mindestens ein Polyol bzw. dessen Methyl- oder Ethylether in wäßriger Lösung enthält und deren pH-Wert zwischen etwa 4,5 und 6,5 beträgt, und die Komponente B Ammoniumhydrogencarbonat, Ammoniumcarbonat und/oder Ammoniumcarbamat enthält und deren pH-Wert zwischen etwa 8 und 9,5 liegt.

Als keratinreduzierende Verbindung können insbesondere Thioglykolsäure oder deren Salze, Thiomilchsäure oder deren Salze, 2-Hydroxy-3-mercaptopropionsäure oder deren Salze, Cystein oder dessen Salze sowie Cysteamin oder dessen Salze, alleine oder in Kombination, verwendet werden.

Die keratinreduzierende Verbindung ist in der gebrauchsfertigen Zubereitung vorzugsweise in einer Menge von 5 bis 20 Gewichtsprozent enthalten, wobei eine Menge von 8 bis 16 Gewichtsprozent besonders bevorzugt ist.

Das in dem erfindungsgemäßen Mittel verwendete Puffersystem besteht vorzugsweise aus Ammoniak, Ammoniumhydrogencarbonat, Ammoniumcarbamat oder Mischungen dieser Verbindungen.

Das Puffersystem weist vorzugsweise einen pH-Wert von 8,2 bis 9,5 auf, wobei ein pH-Wert von 8,5 bis 9 besonders bevorzugt ist.

Die verwendete Menge an Puffersystem ist abhängig von der eingesetzten Menge an keratinreduzierender Verbindung, dem pH-Wert der Komponente A sowie dem für das gebrauchsfertige Haarverformungsmittel gewünschten pH-Wert. In der Regel enthält die Komponente B etwa 1,5 bis 8 Gewichtsprozent des Puffersystems, wobei eine Menge von 1 bis 5 Gewichtsprozent bevorzugt ist.

Das gebrauchsfertige Verformungsmittel wird vor Gebrauch durch Vermischen der beiden Komponenten A und B hergestellt und kann sowohl in Form einer wäßrigen Lösung oder einer Emulsion als auch in verdickter Form auf wäßriger Basis, insbesondere als Gel, Creme oder Paste, vorliegen.

Der pH-Wert dieses Mittels beträgt 7 bis 7,5, wobei ein pH-Wert von 7,1 bis 7,5 bevorzugt ist.

Selbstverständlich kann das Verformungsmittel alle für derartige Mittel üblichen und bekannten Zusatzstoffe, zum Beispiel Verdickungsmittel, wie beispielsweise Kaolin, Bentonit, Fettsäuren, höhere Fettalkohole, Stärke, Polyacrylsäure und deren Derivate, Cellulosederivate, Alginate, Vaseline oder Paraffinöl; Netzmittel oder Emulgatoren aus den Klassen der anionischen, kationischen amphoteren oder nichtionogenen oberflächenaktiven Substanzen, beispielsweise Fettalkoholsulfate, Fettalkoholethersulfate, Alkylsulfonate, Alkylbenzolsulfate, quaternäre Ammoniumsalze, Alkylbetaine, oxethylierte Alkylphenole, Fettsäurealkanolamide, oxethylierte Fettsäureester, Eiweiß-Fettsäure-Kondensationsprodukte, oxethylierte Fettalkohole, Aminoxide oder Alkylpolyglykoside;ferner Trübungsmittel, wie zum Beispiel Polyethylenglykolester oder Vinylpyrrolidon/Styrol- Mischpolymerisate; oder Alkohole, wie beispielsweise Ethanol, Propanol, Isopropanol oder Glycerin; Lösungsvermittler; Stabilisatoren; Puffersubstanzen; Komplexbildner; Parfümöle; Farbstoffe sowie haarkonditionierende und haarpflegende Bestandteile, wie zum Beispiel kationische Polymere, Lanolinderivate, Cholesterin, Pantothensäure oder Betain, enthalten. Die erwähnten Bestandteile werden in den für solche Zwecke üblichen Mengen verwendet, zum Beispiel die Netzmittel und Emulgatoren in Konzentrationen von 0,1 bis 30 Gewichtsprozent, vorzugsweise von 0,1 bis 8 Gewichtsprozent, während die Verdickungsmittel in einer Menge von 0,5 bis 20 Gewichtsprozent in diesem Mittel enthalten sein können.

Weiterhin können dem erfindungsgemäßen Verformungsmittel zur Vermeidung einer Überkrausung der Haare Dithioverbindungen, beispielsweise Dithiodiglykolsäure, Dithiodimilchsäure oder deren Salze, zugesetzt werden.

Das erfindungsgemäße Verformungsmittel ist vorzugsweise frei von sogenannten Quell- und Penetrationsstoffen, wie zum Beispiel Harnstoff, $C_3$- bis $C_6$-Alkandiolen oder deren Ethern, 2-Pyrrolidon, Imidazolidin-2-on oder N-Alkylpyrrolidonen.

Vorzugsweise sind die vorstehend genannten Zusatzstoffe in der das Puffersystem enthaltenden Komponente B enthalten, während die Komponente A die keratinreduzierende Verbindung enthält.

Das erfindungsgemäße Haarverformungsmittel ermöglicht eine schonende und intensive Umformung der Haare.

Die vorliegende Erfindung betrifft daher weiterhin ein Verfahren zur dauerhaften Verformung von Haaren, bei dem man das Haar bevor und/oder nachdem man es in die gewünschte Form bringt, mit einem Verformungsmittel behandelt, mit Wasser spült, sodann oxidativ nachbehandelt, mit Wasser spült, gegebenenfalls zur Wasserwelle legt und sodann trocknet, welches dadurch gekennzeichnet ist, daß als Verformungsmittel das vorstehend beschriebene erfindungsgemäße Mittel verwendet wird.

Bei einer bevorzugten Ausführungsform des erfindungsgemäßen Verfahrens wird das Haar zunächst mit einem Shampoo gewaschen und danach mit Wasser gespült. Anschließend wird das handtuchtrockene Haar in einzelne Strähnen aufgeteilt und auf Wickler mit einem Durchmesser von 5 bis 30 Millimetern, vorzugsweise 5 bis 15 Millimetern, gewickelt. Sodann wird das Haar mit einer für die Haarverformung ausreichenden Menge, vorzugsweise 60 bis 120 Gramm, des beschriebenen erfindungsgemäßen Verformungsmittels behandelt.

Nach einer für die dauerhafte Verformung des Haares ausreichenden Einwirkungszeit, welche je nach Haarbeschaffenheit, dem pH-Wert und der Verformungswirksamkeit des Verformungsmittels sowie in Abhängigkeit von der Anwendungstemperatur 5 bis 30 Minuten (10 bis 30 Minuten ohne Wärmeeinwirkung; 5 bis 20 Minuten mit Wärmeeinwirkung) beträgt, - wobei aufgrund der besseren Haarschonung eine Verformungsbehandlung ohne Wärmeeinwirkung bevorzugt ist - wird das Haar mit Wasser gespült und dann oxidativ nachbehandelt ("fixiert"). Das Nachbehandlungsmittel wird, je nach Haarfülle, vorzugsweise in einer Menge von 80 bis 100 Gramm, verwendet.

Für die oxidative Nachbehandlung kann jedes beliebige, bisher für eine derartige Behandlung verwendete, Nachbehandlungsmittel verwendet werden. Beispiele für in solchen Nachbehandlungsmitteln verwendbare Oxidationsmittel sind Kalium- und Natriumbromat, Natriumperborat, Harnstoffperoxid und Hydrogenperoxid, wobei die Verwendung von Hydrogenperoxid bevorzugt ist.

Die Konzentration des Oxidationsmittels ist in Abhängigkeit von der Anwendungszeit (in der Regel 5 bis 15 Minuten) und der Anwendungstemperatur unterschiedlich. Normalerweise liegt das Oxidationsmittel in dem gebrauchsfertigen wäßrigen Nachbehandlungsmittel in einer Konzentration von 0,5 bis 10 Gewichtsprozent vor. Das Mittel für die oxidative Nachbehandlung kann selbstverständlich weitere Stoffe, wie zum Beispiel Netzmittel, Pflegestoffe wie kationische Polymere und kationische Tenside, schwache Säuren, Puffersubstanzen oder Peroxidstabilisatoren, enthalten und in Form einer wäßrigen Lösung, einer Emulsion sowie in verdickter Form auf wäßriger Basis, insbesondere als Creme, Gel oder Paste, vorliegen.

Anschließend werden die Wickler entfernt. Falls erforderlich, kann das abgewickelte Haar nun nochmals oxidativ nachbehandelt werden. Sodann wird das Haar mit Wasser gespült, gegebenenfalls zur Wasserwelle gelegt und schließlich getrocknet.

Die nachfolgenden Beispiele sollen den Gegenstand der Erfindung näher erläutern, ohne den Gegenstand auf diese Beispiele zu beschränken.

**Beispiele**

**Beispiel 1: Zweikomponenten-Haarverformungsmittel**

**Komponente A:**

| | |
|---|---|
| 95,6 g | Ammoniumthioglykolat (70%-ige wäßrige Lösung) |
| 4,4 g | Thioglykolsäure (98%-ig) |
| 100,0 g | |

Der pH-Wert der Komponente A beträgt 6,0.

**Komponente B:**

| | |
|---|---|
| 1,5 g | Ammoniumhydrogencarbonat |
| 0,6 g | Ammoniak (25%-ige wäßrige Lösung) |
| 1,5 g | Glycerin-Polyethylenglykolrizinoleat (Cremophor®EL der Firma BASF; Ludwigshafen/BRD) |
| 1,0 g | Poly(dimethyldiallylammoniumchlorid) |
| 1,0 g | Cocobetain |
| 0,8 g | Parfümöl |
| 0,5 g | Vinylpyrrolidon/Styrol-Mischpolymerisat (Antara®430 der GAF Corp.; New York/USA) |
| 93,1 g | Wasser |
| 100,0 g | |

Der pH-Wert der Komponente B beträgt 8,5.

Vor Gebrauch werden 14 g der Komponente A und 66 g der Komponente B miteinander vermischt. Der pH-Wert des so erhaltenen Haarverformungsmittels beträgt 7,3.

Das gewaschene und frottierte Haar wird auf Wickler mit einem Durchmesser von 6 Millimetern gewickelt und sodann mit dem vorstehend beschriebenen Haarverformungsmittel gleichmäßig durchfeuchtet.

Nach einer Einwirkungszeit von 18 Minuten wird das Haar gründlich mit Wasser gespült und sodann mit 80 Gramm einer 3-prozentigen wäßrigen Hydrogenperoxidlösung oxidativ nachbehandelt. Nach Entfernung der Wickler werden die Haare erneut mit Wasser gespült, zur Wasserwelle gelegt und anschließend getrocknet.

Das so behandelte Haar besitzt eine gleichmäßige und lebhafte Krause.

**Beispiel 2: Zweikomponenten-Haarverformungsmittel**

**Komponente A:**

| | |
|---|---|
| 71,5 g | Ammoniumthioglykolat (70%-ige wäßrige Lösung) |
| 28,5 g | Thioglykolsäure (99%-ig) |
| 100,0 g | |

Der pH-Wert der Komponente A beträgt 5,2.

**Komponente B:**

| | |
|---|---|
| 4,5 g | Ammoniak (25%-ige wäßrige Lösung) |
| 2,0 g | Ammoniumcarbamat |
| 1,5 g | Glycerin-Polyethylenglykolrizinoleat (Cremophor®EL der Firma BASF; Ludwigshafen/BRD) |
| 0,8 g | Parfümöl |
| 0,5 g | Vinylpyrrolidon/Styrol-Mischpolymerisat (Antara®430 der GAF Corp.; New York/USA) |
| 0,5 g | Cocoamidopropylbetain |
| 0,3 g | Poly(dimethyldiallylammoniumchlorid) |
| 89,9 g | Wasser |
| 100,0 g | |

Der pH-Wert der Komponente B beträgt 8,8.

Vor Gebrauch werden 18 g der Komponente A und 62 g der Komponente B miteinander vermischt. Der pH-Wert des so erhaltenen Haarverformungsmittels beträgt 7,2.

Die Anwendung des Haarverformungsmittels erfolgt in der in Beispiel 1 beschriebenen Weise, wobei die Einwirkungszeit des Haarverformungsmittels 20 Minuten beträgt.

**Beispiel 3: Zweikomponenten-Haarverformungsmittel**

**Komponente A:**

| | |
|---|---|
| 94,6 g | Ammoniumthioglykolat (70%-ige wäßrige Lösung) |
| 5,4 g | Cysteinhydrochlorid |
| 100,0 g | |

Der pH-Wert der Komponente A beträgt 6,0.

**Komponente B:**

| | |
|---|---|
| 0,8 g | Ammoniak (25%-ige wäßrige Lösung) |
| 0,5 g | Ammoniumhydrogencarbonat |
| 2,0 g | Laurylalkohol, mit 4 Mol Ethylenoxid oxethyliert |
| 1,0 g | Poly(dimethyldiallylammoniumchlorid) |
| 1,0 g | Parfümöl |
| 0,5 g | Vinylpyrrolidon/Styrol-Mischpolymerisat (Antara®430 der GAF Corp.; New York/USA) |
| 0,5 g | Cetyltrimethylammoniumchlorid |
| 93,7 g | Wasser |
| 100,0 g | |

Die Komponente B besitzt einen pH-Wert von 8,5.

Vor Gebrauch werden 15 g der Komponente A und 66 g der Komponente B zu einem gebrauchsfertigen Haarverformungsmittel mit einem pH = 7,5 vermischt.

Die Anwendung dieses Haarverformungsmittels erfolgt in der in Beispiel 1 beschriebenen Weise, wobei die Einwirkungszeit des Haarverformungsmittels 12 Minuten beträgt.

**Beispiel 4: Zweikomponenten-Haarverformungsmittel**

**Komponente A:**

| | |
|---|---|
| 95,6 g | Ammoniumthioglykolat (70%-ige wäßrige Lösung) |
| 4,4 g | Thioglykolsäure (98%-ig) |
| 100,0 g | |

Der pH-Wert der Komponente A beträgt 6,0.

**Komponente B:**

| | |
|---|---|
| 0,5 g | Ammoniak (25%-ige wäßrige Lösung) |
| 0,3 g | Ammoniumhydrogencarbonat |
| 1,5 g | Poly(dimethyldiallylammoniumchlorid) |
| 1,5 g | Laurylalkohol, oxethyliert mit 4 Mol Ethylenoxid |
| 0,8 g | Parfümöl |
| 0,5 g | Vinylpyrrolidon/Styrol-Mischpolymerisat (Antara®430 der GAF Corp.; New York/USA) |
| 0,3 g | Cocoamphoacetat |

| | |
|---|---|
| 94,6 g | Wasser |
| 100,0 g | |

Der pH-Wert der Komponente B beträgt 8,5.

12 Gramm der Komponente A werden mit 67 Gramm der Komponente B zu einem gebrauchsfertigen Haarverformungsmittel vom pH 7,4 vermischt.

Das Haar wird gewaschen, mit einem Handtuch frottiert und auf Wickler mit einem Durchmesser von 8 Millimetern gewickelt. Anschließend wird das Haar mit dem vorstehend beschriebenen Haarverformungsmittel gleichmäßig durchfeuchtet. Nach einer Einwirkungszeit von 15 Minuten wird das Haar mit Wasser gründlich gespült und sodann mit 80 Gramm einer 3-prozentigen wäßrigen Hydrogenperoxid-Lösung oxidativ nachbehandelt. Nach Entfernung der Wickler werden die Haare erneut mit Wasser gespült, zur Wasserwelle gelegt und anschließend getrocknet.

Alle in der vorliegenden Anmeldung aufgeführten Prozentangaben beziehen sich auf Gewichtsprozente, sofern nichts anderes angegeben ist.

**Patentansprüche**

**1.** Mittel zur dauerhaften Verformung von Haaren auf der Basis einer keratinreduzierenden Verbindung, welches frei ist von Mercaptocarbonsäureestern und einen pH-Wert von 7 bis 7,5 aufweist, **dadurch gekennzeichnet, daß** es unmittelbar vor der Anwendung durch Vermischen zweier Komponenten hergestellt wird, wobei die eine Komponente (Komponente A) die keratinreduzierende Verbindung enthält und einen pH-Wert von kleiner 7 aufweist, während die andere Komponente (Komponente B) ein Puffersystem enthält und einen pH-Wert von 8 bis 10 aufweist, wobei ein Mittel ausgenommen ist, bei dem die keratinreduzierende Verbindung Thioglykolsäure und/oder ein Salz derselben ist und gleichzeitig die Komponente A mindestens ein Aminosäurechlorid und mindestens ein Polyol bzw. dessen Methyl- oder Ethylether in wäßriger Lösung enthält und deren pH-Wert zwischen etwa 4,5 und 6,5 beträgt, und die Komponente B Ammoniumhydrogencarbonat, Ammoniumcarbonat und/oder Ammoniumcarbamat enthält und deren pH-Wert zwischen etwa 8 und 9,5 liegt.

**2.** Mittel nach Anspruch 1, **dadurch gekennzeichnet, daß** die keratinreduzierende Verbindung in der gebrauchsfertigen Zubereitung in einer Menge von 5 bis 20 Gewichtsprozent enthalten ist.

**3.** Mittel nach Anspruch 1 oder 2, **dadurch gekennzeichnet, daß** die keratinreduzierende Verbindung ausgewählt ist aus Thioglykolsäure oder deren Salzen, Thiomilchsäure oder deren Salzen, 2-Hydroxy-3-mercaptopropionsäure oder deren Salzen, Cystein oder dessen Salzen und Cysteamin oder dessen Salzen.

4. Mittel nach einem der Ansprüche 1 bis 3, **dadurch gekennzeichnet, daß** das Puffersystem ausgewählt ist aus Ammoniak, Ammoniumhydrogencarbonat, Ammoniumcarbamat und Mischungen dieser Verbindungen.

5. Mittel nach einem der Ansprüche 1 bis 4, **dadurch gekennzeichnet, daß** das Puffersystem einen pH-Wert von 8,5 bis 9 aufweist.

6. Mittel nach einem der Ansprüche 1 bis 5, **dadurch gekennzeichnet, daß** das Verformungsmittel frei von Quell- und Penetrationsstoffen ist.

7. Mittel nach einem der Ansprüche 1 bis 6, **dadurch gekennzeichnet, daß** die Komponente A die keratinreduzierende Verbindung enthält, während die Komponente B das Puffersystem sowie die übrigen Zusatzstoffe enthält.

8. Verfahren zur dauerhaften Verformung von Haaren, bei dem man das Haar bevor und/oder nachdem man es in die gewünschte Form bringt mit einem Verformungsmittel behandelt, mit Wasser spült, sodann oxidativ nachbehandelt, mit Wasser spült, gegebenenfalls zur Wasserwelle legt und sodann trocknet, **dadurch gekennzeichnet, daß** man als Verformungsmittel ein Mittel nach einem der Ansprüche 1 bis 7 verwendet.

9. Verfahren nach Anspruch 8, **dadurch gekennzeichnet, daß** man das Verformungsmittel 5 bis 30 Minuten lang einwirken läßt.

10. Verfahren nach Anspruch 8, **dadurch gekennzeichnet, daß** die Verformungsbehandlung ohne Wärmeeinwirkung durchgeführt wird.

**Claims**

1. Agent for the permanent shaping of hair based on a keratin-reducing compound which is free from mercapto-carboxylic acid esters and has a pH of from 7 to 7.5, characterised in that it is prepared immediately prior to use by mixing two components, one component (component A) containing the keratin-reducing compound and having a pH of less than 7, while the other component (component B) contains a buffer system and has a pH of from 8 to 10, with the exclusion of an agent in the case of which the keratin-reducing compound is thioglycolic acid and/or a salt thereof and, at the same time, component A contains at least one amino acid chloride and at least one polyol or its methyl or ethyl ether in aqueous solution and its pH is between approximately 4.5 and 6.5, and component B contains ammonium hydrogen carbonate, ammonium carbonate and/or ammonium carbamate and its pH is between approximately 8 and 9.5.

2. Agent according to Claim 1, characterised in that the amount of keratin-reducing compound present in the ready-for-use preparation is from 5 to 20 % by weight.

3. Agent according to Claim 1 or 2, characterised in that the keratin-reducing compound is selected from thioglycolic acid or its salts, thiolactic acid or its salts, 2-hydroxy-3-mercaptopropionic acid or its salts, cysteine or its salts and cysteamine or its salts.

4. Agent according to any one of Claims 1 to 3, characterised in that the buffer system is selected from ammonia, ammonium hydrogen carbonate, ammonium carbamate and mixtures of those compounds.

5. Agent according to any one of Claims 1 to 4, characterised in that the buffer system has a pH of from 8.5 to 9.

6. Agent according to any one of Claims 1 to 5, characterised in that the shaping agent is free from swelling substances and penetration substances.

7. Agent according to any one of Claims 1 to 6, characterised in that component A contains the keratin-reducing compound while component B contains the buffer system and the remaining additives.

8. Process for the permanent shaping of hair, in which the hair, before and/or after it has been brought into the desired shape, is treated with a shaping agent, rinsed with water, then treated oxidatively, rinsed with water, optionally arranged in a water wave, and then dried, characterised in that an agent according to any one of Claims 1 to 7 is used as the shaping agent.

9. Process according to Claim 8, characterised in that the shaping agent is allowed to take effect for from 5 to 30 minutes.

10. Process according to Claim 8, characterised in that the shaping treatment is carried out without the action of heat.

**Revendications**

1. Produit de mise en forme permanente de cheveux à base d'un composé réducteur de la kératine, exempt d'esters d'acide mercaptocarboxylique et présentant un pH de l'ordre de 7 à 7,5, caractérisé en ce qu'il est préparé directement avant application par mélange de deux composants, un premier composant (composant A) contenant le composé réducteur de la kératine et présentant un pH inférieur à 7, tandis que l'autre composant (composant B) contient un système tampon et a un pH de l'ordre de 8 à 10, à l'exclusion d'un produit dans lequel le composé réducteur de la kératine est l'acide thioglycolique et/ou un sel de celui-ci et simultanément le composant A contient au moins un chlorure d'acide aminé et au moins un polyol ou son éther méthylique ou éthylique en solutin aqueuse dont le pH est situé entre 4,5 et 6,5 environ, et le composant B contient de l'hydrogenocarbonate d'ammonium et/ou du carbonate d'ammonium dont le Ph est situé entre 8 et 9,5 environ.

2. Produit selon la revendication 1, caractérisé en ce que le composé réduceur de la kératine est contenu dans la préparation prête à l'emploi en une quantité de 5 à 20 % en poids.

3. Produit selon la revendication 1 ou 2, caractérisé en ce que le composé réducteur de la kératine est sélectionné entre l'acide thioglycolique ou ses sels, l'acide thiolactique ou ses sels, l'acide 2-hydroxy-3 mercaptopropionique ou ses sels, la cystéine ou ses sels, et la cystéamine ou ses sels.

4. Produit selon l'une des revendications 1 à 3, caractérisé en ce que le système tampon est sélectionné parmi l'ammoniac, l'hydrogénocarbonate d'ammonium, le carbamate d'ammonium et les mélanges de ces composés.

5. Produit selon l'une des revendications 1 à 4, caractérisé en ce que le système tampon présente un pH de l'ordre de 8,5 à 9.

6. Produit selon l'une des revendications 1 à 5, caractérisé en ce qu'il est exempt de produit gonflant et de pénétration.

7. Produit selon l'une des revendications 1 à 6, caractérisé en ce que le composant A contient le composé réducteur de la kératine, tandis que le composant B contient le système tampon ainsi que le reste des additifs.

8. Procédé de mise en forme permanente de cheveux, dans lequel on traite avec un produit de mise en forme, le cheveu avant et/ou après l'avoir mis à la forme souhaitée, on rince à l'eau, puis on effectue un post-traitement oxydant, on rince à l'eau, le cas échéant on expose à une mise en plis et ensuite l'on sèche, caractérisé en ce que l'on utilise comme produit de déformation un produit selon l'une des revendications 1 à 7.

9. Procédé selon la revendication 8, caractérisé en ce que l'on laisse agir le produit de déformation pendant 5 à 30 minutes.

10. Procédé selon la revendication 8, caractérisé en ce que le traitement de déformation a été effectué sans action de chaleur.